# EUROPÄISCHE PATENTSCHRIFT

(11) **EP 2 962 711 B1**
(45) Veröffentlichungstag und Bekanntmachung des Hinweises auf die Patenterteilung: **07.08.2019**
(21) Anmeldenummer: 15172041.4
(22) Anmeldetag: 15.06.2015
(51) Int. Cl.: A61M 1/16

(54) **DIALYSEMASCHINE**
DIALYSIS MACHINE
MACHINE DE DIALYSE

(30) Priorität: 04.07.2014 DE 102014109369
(43) Veröffentlichungstag der Anmeldung: 06.01.2016
(73) Patentinhaber: B. Braun Avitum AG, 34212 Melsungen (DE)
(72) Erfinder: Ritter, Kai-Uwe, 91126 Rednitzhembach (DE)
(74) Vertreter: Winter, Brandl, Fürniss, Hübner, Röss, Kaiser, Polte - Partnerschaft mbB

(56) Entgegenhaltungen:
- WO-A1-2013/141896
- CN-A- 101 564 560
- DE-A1- 2 238 520
- DE-A1- 2 745 572
- US-B2- 6 787 032

## Beschreibung

### Technischer Hintergrund

Die Erfindung betrifft Dialysemaschinen, mit denen Blut eines Patienten gereinigt werden kann, wenn z.B. dessen Nierenfunktion eingeschränkt oder eingestellt ist. Dazu hat die Dialysemaschine einen Dialysator, der einerseits von dem zu reinigenden Blut des Patienten und andererseits von einer Dialysierflüssigkeit, vorzugsweise gemäß dem Gegenstromprinzip durchströmt wird, wobei gewisse gelöste Substanzen (z.B. Harnstoff) aus dem Blut an die Dialysierflüssigkeit übergehen.

Die Erfindung betrifft insbesondere die Aufbereitung der Dialysierflüssigkeit, bevor diese dem Dialysator zugeführt wird. Dabei wird Wasser vorzugsweise aus einer zentralen Osmose-Anlage zunächst entgast und dann werden Konzentrate (z.B. Bikarbonat und saures Konzentrat) zugegeben. Die Konzentrate werden in unterschiedlichen Formen zur Verfügung gestellt, insbesondere in Kanistern, aus denen das jeweilige Konzentrat über Ansaugstäbe angesaugt werden muss, oder über eine zentrale Konzentratversorgung (ZKV), deren Versorgungsleitungen unter Überdruck stehen. Das Bikarbonat kann auch in Pulverform in Kartuschen zur Verfügung gestellt werden. Vor der Zugabe in die Dialysierflüssigkeit wird Wasser durch die Kartusche gepresst, um eine gesättigte Bikarbonat-Lösung herzustellen.

Nach der Zugabe jedes Konzentrats wird die Dialysierflüssigkeit durch einen jeweiligen Leitfähigkeitsmesser geführt, um die Konzentration des jeweiligen Konzentrats in der Dialysierflüssigkeit zu erfassen und um dessen (weitere) Zugabe zu regeln.

### Stand der Technik

Zur Einstellung der jeweiligen Zugabemenge von Konzentraten sind aus dem Stand der Technik volumetrische Pumpen bekannt, die in einer jeweiligen Konzentrat-Zuführleitung angeordnet sind, die in eine zentrale, die Dialysierflüssigkeit führende Hauptleitung der Aufbereitungsanlage münden.

Nachteilig daran ist, dass die heute verwendeten Pumpen teuer sind, während ihre Lebensdauer durch Verschleiß begrenzt ist. Weiterhin besteht das Problem, dass die Konzentrate auskristallisieren können und somit die Pumpe zum Klemmen bringen. Bei der Verwendung von Bikarbonat-Kartuschen dauert ein Befüllen und Entleeren lange. Während einer Desinfektion müssen die Pumpen mit maximaler Geschwindigkeit laufen, um die Zuführleitung mit maximalem Fluss zu durchspülen, was zu erhöhtem Verschleiß und unangenehmer Geräuschbelästigung führt.

Wenn Drehschieberkolbenpumpen zum Einsatz kommen, können durch Kristalle des Konzentrats Undichtigkeiten der Pumpe auftreten. Drehschieberkolbenpumpen erfordern zusätzliche Spülleitungen, die vorrichtungstechnisch aufwändig und damit kostenintensiv sind und zudem desinfiziert werden müssen. Bei der zentralen Konzentratversorgung zeigen Drehschieberkolbenpumpen Undichtigkeiten, da ihr Eingang dauerhaft unter Druck steht.

Die US 6,787,032 B2 offenbart eine Vorrichtung zur Herstellung von Dialyseflüssigkeit, die einen konzentrierten Konzentrattank aufweist, der unter dem Behälterhalter vorgesehen ist. Konzentrat wird über ein Ventil der Hauptleitung zugegeben.

Die DE 2 238 520 offenbart ein Verfahren und eine Vorrichtung zur Herstellung von Hämodialyseflüssigkeit. Dabei wird in einer ersten Ausführungsform das Konzentrat durch eine der Leitungen zugeführt, die ein elektromagnetisch gesteuertes Ventil enthalten und mit einer Einspritzvorrichtung verbunden sind. Um jeweils ein Element als Reserve zur Verfügung zu haben sind die Zuführleitungen mit den Ventilen jeweils paarweise vorgesehen.

Aus der DE 27 45 572 ist eine Aufbereitungsanlage für Dialysierflüssigkeit bekannt, bei der in der Konzentrat-Zuführleitung eines Salzlösungskonzentrats, die in die Hauptleitung für die Dialysierflüssigkeit mündet, ein Zuführ-Drosselventil angeordnet ist. Das Zuführ-Drosselventil wird über einen Regler in Abhängigkeit eines

Konduktivitätsmessers gesteuert, der die Konzentration der Salzlösung in der Dialysierflüssigkeit bestimmt. In der Hauptleitung folgen dabei - in Strömungsrichtung der Dialysierflüssigkeit betrachtet - zunächst ein Haupt-Drosselventil, dann eine Mündung der Konzentrat-Zuführleitung und dann eine verstellbare Pumpe, die in Abhängigkeit eines im Bereich der Mündung angeordneten Druckfühlers von einem Druckregler geregelt wird.

Für die heute üblichen Dialysierflüssigkeits-Flüsse im Bereich von 100 - 1000 ml/min ergibt sich mit der Lösung gemäß der DE 27 45 572 eine sehr starke Veränderung des Unterdrucks an der Mündung der Konzentrat-Zuführleitung. Der Unterdruck muss dann derart ausgelegt werden, dass das Konzentrat auch bei 100 ml/min noch sicher angesaugt wird. Dadurch ergeben sich bei den höheren Flüssen von 800 - 1000ml/min sehr hohe Unterdrücke, die zu einer Geräuschbelästigung und zu einer hohen Pumpenleistung führen. Dabei ist auch ein Ausgasen der Dialysierflüssigkeit an der Mündung und in der Pumpe möglich, wobei letzteres zu Kavitationsschäden führt. Die Einstellung bzw. Regelung ist schwierig, da das Zuführ-Drosselventil (Stromregelventil) der Konzentrat-Zuführleitung eine starke Regelsteilheit bekommt.

Nachteilig an einer derartigen Aufbereitungsanlage für Dialysierflüssigkeit ist, dass stromab des Haupt-Drosselventils von der Pumpe ein stark flussabhängiger Unterdruck in der Hauptleitung erzeugt wird. Der Druck an der Mündung der Konzentrat-Zuführleitung soll über die Pumpendrehzahl der Pumpe geregelt werden. Dies funktioniert nur für einen bestimmten Fluss. Fluss und Unterdruck lassen sich bei dieser Aufbereitungsanlage nicht getrennt einstellen.

### Kurze Beschreibung der Erfindung

Dem gegenüber liegt der Erfindung die Aufgabe zu Grunde, eine Dialysemaschine zu schaffen, bei der die genannten Nachteile vermieden sind.

Diese Aufgabe wird gelöst durch eine Dialysemaschine mit den Merkmalen des Patentanspruchs 1. Weitere vorteilhafte Ausgestaltungen der Erfindung sind in den abhängigen Patentansprüchen beschrieben.

Die beanspruchte Dialysemaschine weist gemäß einem Aspekt der Erfindung eine Aufbereitungsanlage mit einer Hauptleitung für Dialysierflüssigkeit auf, an die eine oder mehrere Zuführleitungen für jeweilige der Dialysierflüssigkeit zuzugebende Flüssigkeiten (meistens Konzentrate) münden, wobei hinter (stromab) den Mündungen eine (Saug-)Pumpe vorgesehen ist. Damit wird ein Unterdruck (gegenüber der Atmosphäre) in der Hauptleitung erzeugt, auf Grund dessen die Konzentrate in die Hauptleitung gesaugt werden. Erfindungsgemäß ist vor (stromauf) einer ersten Mündung ein elektromagnetisch oder vorzugsweise ein druckbetätigtes sowie weiter vorzugsweise einstellbares angesteuertes Druckregelventil in der Hauptleitung angeordnet und in jeder Zuführleitung ist eine elektromagnetisch angesteuerte Ventilanordnung (Schaltventil) vorgesehen. Damit kann auch über einen großen Bereich an verschiedenen Flussgeschwindigkeiten der Dialysierflüssigkeit stets ein minimal nötiger Unterdruck in der Hauptleitung eingeregelt werden, wodurch die Regelbarkeit der Konzentrationen verbessert ist. Weiterhin wird die Pumpe nicht unnötig belastet.

Trotz stark unterschiedlicher (einstellbarer) Flussgeschwindigkeiten kann mit der erfindungsgemäßen Dialysemaschine z.B. gegenüber der Atmosphäre ein Unterdruck von -200mbar in der Hauptleitung eingeregelt werden, der ausreicht, um z.B. ein Konzentrat aus einem Kanister mit einer Zuführleitung, die einen Durchmesser von vorzugsweise 3 mm hat, über einen Höhenunterschied von bis zu 70 cm in die Hauptleitung zu saugen. Im Falle einer Bikarbonat-Kartusche kann diese durch vollständiges Öffnen der zugeordneten Ventilanordnung schnell entleert und gefüllt werden. Bei einer Desinfektion der erfindungsgemäßen Dialysemaschine kann die zumindest eine Ventilanordnung maximal (bzw. dauerhaft) geöffnet werden.

Besonders bevorzugt ist eine elektronische Steuereinheit, über die einerseits vorzugsweise das Druckregelventil einstellbar ist und über die andererseits die zumindest eine Ventilanordnung in Abhängigkeit einer ihr zugeordneten Leitfähigkeitsmessvorrichtung einstellbar ist. Diese kann eine Leitfähigkeitssonde sein, welche vorzugsweise in der Hauptleitung angeordnet ist.

Damit ein Ausgangssignal der Leitfähigkeitsmessvorrichtung vergleichbar ist, auch wenn die zugeordneten Ventilanordnung das Konzentrat unstetig oder sprunghaft dosiert, ist es besonders bevorzugt, eine Mischkammer zwischen der zugeordneten Mündung und der zugeordneten Leitfähigkeitsmessvorrichtung anzuordnen.

Die zumindest eine Ventilanordnung kann ein Proportionalventil sein. Damit kann der zugeführte Fluss des Konzentrats über dessen Öffnungsquerschnitt (kontinuierlich) eingestellt werden.

Alternativ kann die zumindest eine Ventilanordnung ein erstes digitales (elektromagnetisch betätigtes) Schaltventil (2/2-Schaltventil) aufweisen. Dann kann der zugeführte Fluss des Konzentrats über verschiedene, insbesondere periodisch wiederkehrende Öffnungsimpulse eingestellt werden (amplituden-, pulsweiten- oder frequenzmoduliert).

Bei der Weiterbildung der erfindungsgemäßen Dialysemaschine mit der Mischkammer wird eine Frequenz und / oder Pulsweite der Öffnungsimpulse des zugeordneten Schaltventils vorzugsweise derart gewählt, dass während einer Befüllung der Mischkammer mehrere Öffnungsvorgänge des Schaltventils erfolgen. Dadurch wird sichergestellt, dass die Mischkammer die Dialysierflüssigkeit und das Konzentrat optimal mischen bzw. aufbereiten kann.

Bei einer bevorzugten Ausgestaltung sind an die Hauptleitung eine erste Zuführleitung mit einem ersten Schaltventil und eine zweite Zuführleitung mit einem zweiten Schaltventil angeschlossen, wobei die beiden Schaltventile vorzugsweise gleiche oder unterschiedliche Öffnungsquerschnitte haben.

Dann kann an der ersten Zuführleitung Bikarbonat aus einem Kanister und an der zweiten Zuführleitung saures Konzentrat aus einem weiteren Kanister bereitgestellt werden. In diesem Fall muss die Dialysemaschine erkennen können, wenn Kanister vertauscht werden. Würden nämlich fälschlicherweise zwei Bikarbonat-Kanister angeschlossen werden, könnte das zu einer Alkalose des Patienten führen. Diese Gefahr kann dadurch angegangen werden, dass zu Beginn einer Therapie ein Ventil in der Strecke der Dialyseflüssigkeitaufbereitung geschlossen wird und nur Konzentrate ohne Wasser angesaugt werden, so dass durch Prüfung der Leitfähigkeit unerwünschte Flüssigkeiten ausgeschlossen werden können.

Aufgrund der Leitfähigkeitsverhältnisse muss die Öffnungszeit des ersten Schaltventils deutlich länger als die des zweiten Schaltventils sein. Dieses Verhältnis sowie ein zulässiger Bereich der Öffnungszeiten kann von der elektronischen Regel-/Steuereinheit geprüft werden.

Wenn an der ersten Zuführleitung Bikarbonat aus einem Kanister und an der zweiten Zuführleitung saures Konzentrat aus einer zentralen Konzentratversorgung bereitgestellt werden, dann muss die zentrale Konzentratversorgung nicht überwacht werden. Jedoch kann als Sicherheitsmaßnahme sichergestellt werden, dass ein zulässiger Bereich der Öffnungszeiten des ersten Schaltventils nicht überschritten wird.

Wenn das Bikarbonat und das saure Konzentrat beide über die zentrale Konzentratversorgung bereitgestellt werden, ist keine Prüfung erforderlich.

Wenn das Bikarbonat aus einer Kartusche und das saure Konzentrat aus einem Kanister bereitgestellt werden, dann muss die zentrale Konzentratversorgung nicht überwacht werden. Jedoch kann in diesem Fall als Sicherheitsmaßnahme sichergestellt werden, dass ein zulässiger Bereich der Öffnungszeiten des zweiten Schaltventils nicht überschritten wird.

Damit lässt sich eine falsche Dosierung, insbesondere des Bikarbonats, durch Vertauschen von Kanistern verhindern.

Die zumindest eine Ventilanordnung hat ein weiteres digitales Schaltventil, das parallel zum ersten Schaltventil geschaltet ist. Dann sind bei gleichen Öffnungsquerschnitten zwei und bei ungleichen Öffnungsquerschnitten drei verschiedene dauerhafte bzw. nominelle Öffnungsquerschnitte der Ventilanordnung realisierbar. Damit kann z.B. bei einer Ansaugung des Konzentrats aus einem Kanister ein großer Öffnungsquerschnitt gewählt werden, und bei einer Zuführung des Konzentrats aus der zentralen Konzentratversorgung wird wegen deren Überdruck ein kleiner Öffnungsquerschnitt gewählt.

Darüber hinausgehend kann natürlich auch bei dieser Weiterbildung der zugeführte Fluss des Konzentrats über aufeinander folgende Öffnungsimpulse eingestellt werden.

Wenn die Öffnungsquerschnitte der beiden Schaltventile der jeweils einen Ventilanordnung ein Verhältnis von 1 zu 2 haben, sind die Abstufungsmöglichkeiten der Ventilanordnung optimiert. Es können auch mehr als zwei parallele Schaltventile pro Ventilanordnung vorgesehen sein, deren Öffnungsquerschnitte dann vorzugsweise im Verhältnis 1 : 2 : 4: 8 : 16 usw. gestuft sind.

Bei der Weiterbildung mit nur einem Schaltventil pro Ventilanordnung und bei der Weiterbildung mit mehreren Schaltventilen pro Ventilanordnung kann eine Dosierung des Konzentrats dadurch erfolgen, dass bei einer gleichbleibenden Frequenz unterschiedlich lange Öffnungsimpulse von der Steuereinheit übertragen werden (pulsweitenmoduliertes Verfahren), oder dass gleichlange Öffnungsimpulse mit unterschiedlicher Frequenz übertragen werden (pulsfrequenzmoduliertes Verfahren).

Ein vorteilhafter Betrieb ist bei einem Mischkammervolumen von 100ml eine Dosierung mit 3-5 Öffnungsvorgängen pro 100ml. Bei einer angenommenen Lebensdauer von 20.000h für ein Ventil ergeben sich je nach Fluss der Dialysierflüssigkeit 30-50 Mio. Schaltspiele des zugeordneten Schaltventils. Häufigere Öffnungsvorgänge sind vorteilhaft, falls Ventile mit entsprechender Lebensdauer zur Verfügung stehen. Weniger Öffnungen erhöhen die Pulsation der Leitfähigkeit der Dialysierflüssigkeit, so dass eine ausreichende Glättung der Leitfähigkeit erschwert wird. Die Öffnungsfrequenz ist abhängig vom Fluss, so werden bei einem Fluss von 100ml/Min 4 Öffnungen/Min und bei einem Fluss von 500ml/Min 20 Öffnungen/Min und bei einem Fluss von 800ml/Min 32 Öffnungen/Min vorgeschlagen.

Um eine besonders hohe Fehlersicherheit der erfindungsgemäßen Dialysemaschine zu erreichen, wird vorzugsweise in der Hauptleitung zwischen der letzten Leitfähigkeitsmessvorrichtung und der Pumpe eine abschließende Leitfähigkeitsmessvorrichtung angeordnet, die mit einer Überwachungseinrichtung verbunden ist. Die Leitfähigkeitsmessvorrichtung kann eine Leitfähigkeitssonde sein.

Bei einer bevorzugten Weiterbildung der erfindungsgemäßen Dialysemaschine ist in der Hauptleitung vor (stromauf) den Mündungen der Zulaufleitungen ein elektromagnetisch angesteuertes Absperrventil angeordnet. Damit kann ein Test durchgeführt werden, bei dem ein eventuell falsches Konzentrat bzw. ein falscher Kanister erkannt wird. Dazu wird das Absperrventil geschlossen, so dass von der Pumpe kein Wasser sondern nur das (zumindest eine) Konzentrat angesaugt wird. Eine Messung der Leitfähigkeit über die (zumindest eine) zugeordnete Leitfähigkeitsmessvorrichtung kann mit (zumindest einem) hinterlegten Wert abgeglichen werden.

### Ausführungsbeispiele

Im Folgenden wird anhand der Figuren ein Ausführungsbeispiel der Erfindung detailliert beschrieben. Es zeigen:
Figur 1 ein Ausführungsbeispiel der erfindungsgemäßen Dialysemaschine;
Figur 2 schematische Diagramme mit verschiedenen Leitfähigkeiten einer Dialysierflüssigkeit der Dialysemaschine gemäß Figur 1;
Figur 3 ein Diagramm mit einem Signal eines Schaltventils und einer Leitfähigkeit vor einer zugeordneten Mischkammer der Dialysemaschine gemäß Figur 1; und
Figur 4 ein Diagramm mit einem Signal eines Schaltventils und einer Leitfähigkeit nach einer zugeordneten Mischkammer der Dialysemaschine gemäß Figur 1.

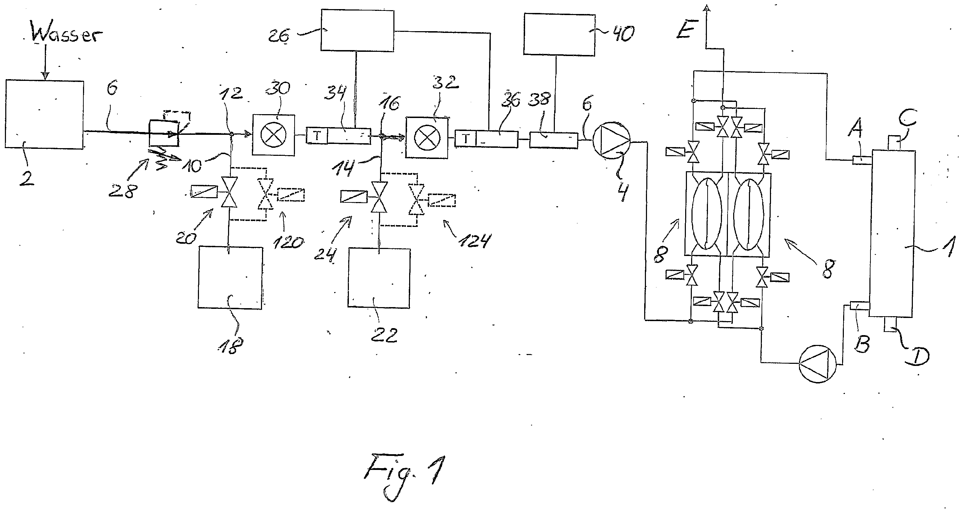

Figur 1 zeigt einen Schaltplan eines Ausführungsbeispiels einer erfindungsgemäßen Dialysemaschine. Sie hat einen Dialysator 1, der einerseits über Anschlüsse A, B von einer Dialysierflüssigkeit und andererseits über Anschlüsse C, D von dem zu reinigenden Blut eines Patienten durchströmt wird. Die Dialysemaschine hat ferner eine Aufbereitungsanlage für die Dialysierflüssigkeit. Von einer Wasserversorgung wird Wasser in einen Tank 2 geleitet, in dem eine Entgasung stattfindet. Von dort wird das Wasser bzw. die Dialysierflüssigkeit von einer Pumpe 4 durch eine Hauptleitung 6 gesaugt und danach durch ein System mit zwei Bilanzierungskammern 8 gefördert. Jede Bilanzierungskammer 8 hat eine Verbindung mit der Hauptleitung 6 für frisch aufbereitete Dialysierflüssigkeit, eine Verbindung mit dem Anschluss A des Dialysators 1, eine Verbindung mit dem Anschluss B des Dialysators 1 und eine Verbindung mit einem Ablauf E für verbrauchte Dialysierflüssigkeit. Jede Bilanzierungskammer 8 ist durch eine Membran in zwei Kammerabschnitte getrennt. Das System mit den beiden Bilanzierungskammern 8 stellt sicher, dass die dem Dialysator 1 zuströmende Menge gleich der vom Dialysator 1 abströmenden Menge ist.

Bei dem gezeigten Ausführungsbeispiel der Aufbereitungsanlage wird dem Wasser über eine erste Zuführleitung 10, die an einer ersten Mündung 12 in die Hauptleitung 6 mündet, ein erstes Konzentrat aus Bikarbonat zugeführt. Dahinter wird der Dialysierflüssigkeit über eine zweite Zuführleitung 14, die an einer zweiten Mündung 16 in die Hauptleitung 6 mündet, ein zweites saures Konzentrat zugeführt.

In der ersten Zuführleitung 10, die in einen ersten Kanister 18 eingetaucht ist, ist ein erstes digitales Schaltventil 20 angeordnet, während in der zweiten Zuführleitung 14, die in einen zweiten Kanister 22 eingetaucht ist, ein zweites digitales Schaltventil 24 angeordnet ist. Beide Schaltventile sind über einen jeweiligen elektromagnetischen Aktor von einer elektronischen Steuereinheit 26 in eine Öffnungsstellung schaltbar.

Zwischen der ersten Mündung 12 und dem Tank 2 ist in der Hauptleitung 6 ein Druckregelventil 28 angeordnet. Das Druckregelventil 28 kann in einer bevorzugten Ausgestaltung ebenfalls elektromagnetisch von der Steuereinheit 26 angesteuert werden. Damit wird der von der Pumpe 4 erzeugte Unterdruck in der Hauptleitung 6 so geregelt, dass nur der zum Ansaugen der Konzentrate notwendige Unterdruck (z.B. - 200 mbar) an den beiden Mündungen 12, 16 herrscht.

Nach jeder Mündung 12, 16 ist eine der Mündung zugeordnete Mischkammer 30, 32 und jeweils dahinter eine der Mündung zugeordnete Leitfähigkeitssonde 34, 36 angeordnet.

Im Betrieb der erfindungsgemäßen Dialysemaschine gibt die Steuereinheit 26 Öffnungsimpulse nach dem pulsweitenmodulierten Verfahren oder nach dem pulsfrequenzmodulierten Verfahren auf den jeweiligen elektromagnetischen Aktor des Schaltventile 20, 24. Dabei wird die aufsummierte Öffnungszeit des jeweiligen Schaltventils 20, 24 in Abhängigkeit eines Signals der zugeordneten Leitfähigkeitssonde 34, 36 geregelt, über die die Konzentration des zugeordneten Konzentrats ermittelt wird.

Zwischen der zweiten Leitfähigkeitssonde 36 und der Pumpe 4 ist eine weitere Leitfähigkeitssonde 38 angeordnet, die ihr Signal an eine abschließende elektronische Überwachungseinrichtung 40 zur Überprüfung der Leitfähigkeit der fertig aufbereiteten Dialyseflüssigkeit überträgt.

Bei der erfindungsgemäßen Dialysemaschine ist parallel zu jedem Schaltventil 20, 24 ein jeweiliges (in Figur 1 gestrichelt dargestelltes) weiteres Schaltventil 120, 124 vorgesehen. Damit können, insbesondere wenn die beiden parallel zueinander geschalteten Schaltventile verschiedene Öffnungsquerschnitte haben, verschiedene nominelle Öffnungsquerschnitte der so gebildeten Ventilanordnung realisiert werden.

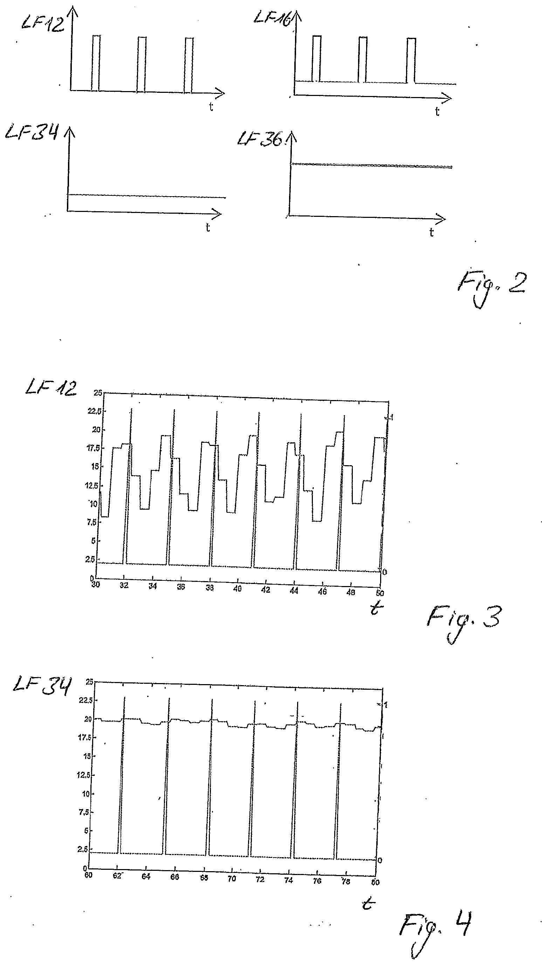

Figur 2 zeigt in vier Diagrammen schematisch die zu erwartenden Leitfähigkeiten der Dialysierflüssigkeit über die Zeit t an vier Stellen der Hauptleitung 6. Genauer gesagt hat die Dialysierflüssigkeit die sprunghafte Leitfähigkeit LF12 nach der ersten Mündung 12, also nachdem das Bikarbonat-Konzentrat durch Öffnungsimpulse des ersten Schaltventils 20 zugegeben wurde. Hinter der ersten Mischkammer 30 wird von der ersten Leitfähigkeitssonde 34 die geglättete Leitfähigkeit LF34 ermittelt. Nach der zweiten Mündung 16, also nachdem das saure Konzentrat durch Öffnungsimpulse des zweiten Schaltventils 24 zugegeben wurde, hat die Dialysierflüssigkeit die sprunghafte Leitfähigkeit LF16. Hinter der zweiten Mischkammer 32 wird von der zweiten Leitfähigkeitssonde 36 die geglättete Leitfähigkeit LF36 ermittelt.

Die über die Zeit konstante Leitfähigkeit LF36 der Dialysierflüssigkeit besteht auch in einer Leitung zwischen der Bilanzierungskammer 8 und dem Anschluss A des Dialysators 1.

Figur 3 zeigt über die Zeit t die periodischen Öffnungsimpulse des ersten Schaltventils 20 für Bikarbonat-Konzentrat und die gemessene unstetige Leitfähigkeit LF12 der Dialysierflüssigkeit hinter der zugeordneten ersten Mündung 12.

Figur 4 zeigt über die Zeit t die periodischen Öffnungsimpulse des ersten Schaltventils 20 für Bikarbonat-Konzentrat und die durch die erste Leitfähigkeitssonde 34 hinter der ersten Mischkammer 30 gemessene Leitfähigkeit LF34 der Dialysierflüssigkeit. Die Vermischung des Bikarbonat-Konzentrats mit dem Wasser in der ersten Mischkammer 30 führt zu einer Glättung des Verlaufs der Leitfähigkeit und kann so als Regelgröße für den Regler der elektronischen Steuereinheit 26 genutzt werden, der nach dem pulsweitenmodulierten Verfahren oder nach dem pulsfrequenzmodulierten Verfahren die Dosierung der Bikarbonat-Konzentration anpasst.

Abweichend vom ersten Ausführungsbeispiel gemäß Figur 1 kann statt dem ersten Kanister 18 eine Bikarbonat-Kartusche oder eine zentrale Konzentratversorgung angeschlossen werden. Statt dem zweiten Kanister 22 kann auch die zentrale Konzentratversorgung angeschlossen werden.

Offenbart ist eine Dialysemaschine, die eine Aufbereitungsanlage mit einer Hauptleitung für Dialyseflüssigkeit aufweist, an der mehrere Zuführleitungen für jeweilige Konzentrate münden. Nach jeder Mündung sind eine jeweilige Mischkammer und danach eine jeweilige Leitfähigkeitssonde angeordnet. Nach der letzten Leitfähigkeitssonde ist eine gemeinsame Pumpe angeordnet. Der Unterdruck in der Hauptleitung wird über ein Druckregelventil eingestellt, das vor der ersten Mündung in der Hauptleitung angeordnet ist. Weiterhin sind zur Dosierung der verschiedenen Konzentrate in jeder Zuführleitung ein Proportionalventil oder ein digitales Schaltventil vorgesehen. Das Druckregelventil und die Proportionalventile bzw. Schaltventile werden elektromagnetisch von einer zentralen elektronischen Steuereinheit gesteuert. Mit anderen Worten wird eine Anordnung zur Dialysierflüssigkeitsaufbereitung bereitgestellt, bei der auf volumetrische Pumpen verzichtet wird und diese durch Ventile ersetzt sind. Bei dieser Anordnung erfolgt die Volumen- bzw. Dosierungsregelung mittels einer Regelschleife über die Leitfähigkeitsmessung. Dabei werden die hydraulischen Verhältnisse im System derart angepasst, dass ein auf diese Weise erzeugter relativer Unterdruck zu einer Umgebung zum Ansaugen einzelner Flüssigkeiten ausreicht. Zudem wird durch eine besondere Anordnung sowie Reihenfolge in der Öffnung der Ventile die Sicherheit gegen eine Behältervertauschung erhöht.

### Bezugszeichenliste

- 1: Dialysator
- 2: Tank
- 4: Pumpe
- 6: Hauptleitung
- 8: Bilanzierungskammer
- 10: erste Zuführleitung
- 12: erste Mündung
- 14: zweite Zuführleitung
- 16: zweite Mündung
- 18: erster Kanister
- 20: erstes Schaltventil
- 22: zweiter Kanister
- 24: zweites Schaltventil
- 26: elektronische Steuereinheit
- 28: Druckregelventil
- 30: erste Mischkammer
- 32: zweite Mischkammer
- 34: erste Leitfähigkeitssonde
- 36: zweite Leitfähigkeitssonde
- 38: weitere Leitfähigkeitssonde
- 40: Überwachungseinrichtung
- 120, 124: weiteres Schaltventil

- A, B, C, D: Anschluss
- E: Ablauf
- LF12: Leitfähigkeit an erster Mündung
- LF34: Leitfähigkeit an erster Leitfähigkeitssonde
- LF16: Leitfähigkeit an zweiter Mündung
- LF36: Leitfähigkeit an zweiter Leitfähigkeitssonde

## Patentansprüche

1. Dialysemaschine mit einer Aufbereitungsanlage für Dialysierflüssigkeit, die eine Hauptleitung (6) für die Dialysierflüssigkeit aufweist, an die über wenigstens eine Mündung (12, 16) wenigstens eine Zuführleitung (10, 14) für wenigstens eine der Dialysierflüssigkeit zuzugebende Flüssigkeit angeschlossen ist, wobei stromab zur wenigstens einen Mündung (12, 16) eine Pumpe (4) in der Hauptleitung (6) vorgesehen ist, wobei vor der wenigstens einen Mündung (12, 16) ein Druckregelventil (28) in der Hauptleitung (6) angeordnet ist, und dass in der wenigstens einen Zuführleitung (10, 14) eine elektromagnetisch angesteuerte Ventilanordnung vorgesehen ist, wobei die jeweilige Ventilanordnung wenigstens zwei parallele Schaltventile (20, 120, 24, 124) umfasst **dadurch gekennzeichnet, dass** die wenigstens zwei parallelen Schaltventile (20, 120, 24, 124) der jeweiligen Ventilanordnung im vollständig geöffneten Zustand ungleiche Öffnungsquerschnitte haben.

2. Dialysemaschine nach Anspruch 1, **gekennzeichnet durch** eine elektronische Regel-/Steuereinheit (26), über die zumindest die Ventilanordnung (20, 120, 24, 124) in Abhängigkeit einer ihr zugeordneten Leitfähigkeitsmessvorrichtung (34, 36) in der Hauptleitung (6) einstellbar ist.

3. Dialysemaschine nach Anspruch 2, **gekennzeichnet durch** eine der Leitfähigkeitsmessvorrichtung (34, 36) zugeordnete Mischkammer (30, 32), die zwischen der Mündung (12, 16) und der zugeordneten Leitfähigkeitsmessvorrichtung (34, 36) angeordnet ist.

4. Dialysemaschine nach einem der vorhergehenden Ansprüche, **dadurch gekennzeichnet, dass** die Ventilanordnung ein Proportionalventil ist.

5. Dialysemaschine nach einem der Ansprüche 1 bis 3, **dadurch gekennzeichnet, dass** die Ventilanordnung ein Schaltventil (20, 24) aufweist.

6. Dialysemaschine nach Anspruch 5, **gekennzeichnet durch** eine erste Zuführleitung (10) mit einem ersten Schaltventil (20) und eine zweite Zuführleitung (14) mit einem zweiten Schaltventil (24), wobei die beiden Schaltventile (20, 24) gleiche oder unterschiedliche Öffnungsquerschnitte haben.

7. Dialysemaschine nach Anspruch 5, **dadurch gekennzeichnet, dass** die Ventilanordnung ein weiteres Schaltventil (120, 124) aufweist, das parallel zum ersten Schaltventil (20, 24) angeordnet ist.

8. Dialysemaschine nach Anspruch 7, **dadurch gekennzeichnet, dass** die Öffnungsquerschnitte der beiden Schaltventile (20, 120, 24, 124) der jeweiligen Ventilanordnung ein Verhältnis von 1 : 2 haben.

9. Dialysemaschine nach einem der Ansprüche 5 bis 8, **dadurch gekennzeichnet, dass** an das Schaltventil (20, 24) oder an die Schaltventile (20, 120, 24, 124) vorzugsweise bei einer gleichbleibenden Frequenz unterschiedlich lange Öffnungsimpulse übertragbar sind.

10. Dialysemaschine nach einem der Ansprüche 5 bis 8, **dadurch gekennzeichnet, dass** an das Schaltventil (20, 24) oder an die Schaltventile (20, 120, 24, 124) gleichlange Öffnungsimpulse mit unterschiedlicher Frequenz übertragbar sind.

11. Dialysemaschine nach einem der Ansprüche 3 bis 10, **dadurch gekennzeichnet, dass** in der Hauptleitung (6) zwischen der Leitfähigkeitsmessvorrichtung (36) und der Pumpe (4) eine weitere Leitfähigkeitsmessvorrichtung (38) angeordnet ist, deren Signal an eine Überwachungseinrichtung (40) übertragbar ist.

12. Dialysemaschine nach einem der vorhergehenden Ansprüche, **dadurch gekennzeichnet, dass** in der Hauptleitung (6) stromauf zur Mündung (12) ein elektromagnetisch angesteuertes Absperrventil angeordnet ist.

13. Dialysemaschine nach Anspruch 1, **dadurch gekennzeichnet, dass** das Druckregelventil (28) ein druckbetätigtes, einstellbares Druckregelventil ist.

14. Dialysemaschine nach Anspruch 5, **dadurch gekennzeichnet, dass** das Schaltventil (20, 24) ein 2/2-Schaltventil ist

15. Dialysemaschine nach Anspruch 7, **dadurch gekennzeichnet, dass** das weitere Schaltventil (120, 124) ein 2/2-Schaltventil ist.

## Claims

1. A dialysis machine comprising a preparation system for a dialysis liquid, comprising a main line (6) for the dialysis liquid to which at least one supply line (10, 14) for at least one liquid to be added to the dialysis liquid is connected via at least one orifice (12, 16), a pump (4) being provided in the main line (6) downstream of the at least one orifice (12, 16), wherein a pressure control valve (28) is arranged in the main line (6) upstream of the at least one orifice (12, 16), and in that an electromagnetically controlled valve assembly is provided in the at least one supply line (10, 14), wherein the respective valve assembly comprises at least two parallel switching valves (20, 120, 24, 124), **characterized in that** the at least two parallel switching valves (20, 120, 24, 124) of the respective valve assembly have different port sizes in a completely opened state.

2. The dialysis machine according to claim 1, ***characterized by*** an electronic regulating/control unit (26) which is able to adjust at least the valve assembly (20, 120, 24, 124) as a function of an associated conductivity measuring device (34, 36) in the main line (6).

3. The dialysis machine according to claim 2, ***characterized by*** a mixing chamber (30, 32) which is associated to the conductivity measuring device (34, 36) and arranged between the orifice (12, 16) and the associated conductivity measuring device (34, 36).

4. The dialysis machine according to any of the preceding claims, ***characterized in that*** the valve assembly is a proportional valve.

5. The dialysis machine according to any of claims 1 to 3, ***characterized in that*** the valve assembly comprises a switching valve (20, 24).

6. The dialysis machine according to claim 5, ***characterized by*** a first supply line (10) comprising a first switching valve (20) and a second supply line (14) comprising a second switching valve (24), the two switching valves (20, 24) having the same or different port sizes.

7. The dialysis machine according to claim 5, ***characterized in that*** the valve assembly comprises a further switching valve (120, 124), which is arranged parallel to the first switching valve (20, 24).

8. The dialysis machine according to claim 7, ***characterized in that*** the port sizes of the two switching valves (20, 120, 24, 124) of the respective valve assembly have a ratio of 1:2.

9. The dialysis machine according to any of claims 5 to 8, ***characterized in that*** opening impulses with differing lengths and preferably with a constant frequency can be transferred to the switching valve (20, 24) or the switching valves (20, 120, 24, 124).

10. The dialysis machine according to any of claims 5 to 8, ***characterized in that*** opening impulses with the same length but with differing frequencies can be transferred to the switching valve (20, 24) or the switching valves (20, 120, 24, 124).

11. The dialysis machine according to any of claims 3 to 10, ***characterized in that*** a further conductivity measuring device (38) whose signal can be transferred to a monitoring equipment (40) is arranged in the main line (6) between the conductivity measuring device (36) and the pump (4).

12. The dialysis machine according to any of the preceding claims, ***characterized in that*** an electromagnetically controlled shutoff valve is arranged in the main line (6) upstream of the orifice (12).

13. The dialysis machine according to claim 1, ***characterized in that** the* pressure control valve (28) is a pressure-operated, adjustable pressure control valve.

14. The dialysis machine according to claim 5, ***characterized in that** the* switching valve (20, 24) is a 2/2-switching valve.

15. The dialysis machine according to claim 7, ***characterized in that*** the further switching valve (120, 124) is a 2/2-switching valve.

## Revendications

1. Machine de dialyse comportant une installation de préparation pour liquide de dialyse qui présente une conduite principale (6) pour le liquide de dialyse, à laquelle par au moins une embouchure (12, 16) au moins une conduite d'acheminement (10, 14) est raccordée pour au moins un liquide apporté au liquide de dialyse, dans laquelle en aval d'au moins une embouchure (12, 16), une pompe (4) est prévue dans la conduite principale (6), dans laquelle avant l'au moins une embouchure (12, 16), une soupape de réglage de la pression (28) est aménagée dans la conduite principale (6), et en ce que dans l'au moins une conduite d'acheminement (10, 14), un système de soupape commandé de façon électromagnétique est prévu, dans laquelle le système de soupape respectif comprend au moins deux soupapes de commutations parallèles (20, 120, 24, 124), **caractérisée en ce que** les au moins deux soupapes de commutation parallèles (20, 120, 24, 124) du système de soupape respectif ont à l'état complètement ouvert, des sections d'ouvertures inégales.

2. Machine de dialyse selon la revendication 1, **caractérisée par** une unité de réglage/commande électronique (26) par laquelle au moins le système de soupape (20, 120, 24, 124) peut être réglé en fonction d'un dispositif de mesure de la conductivité (34, 36) qui lui est affecté dans la conduite principale (6).

3. Machine de dialyse selon la revendication 2, **caractérisée par** une chambre de mélange (30, 32) affectée au dispositif de mesure de la conductivité (34, 36) qui est aménagée entre l'embouchure (12, 16) et le dispositif de mesure de la conductivité (34, 36) affecté.

4. Machine de dialyse selon l'une quelconque des revendications précédentes, **caractérisée en ce que** le système de soupape est une soupape proportionnelle.

5. Machine de dialyse selon l'une quelconque des revendications 1 à 3, **caractérisée en ce que** le système de soupape présente une soupape de commutation (20, 24).

6. Machine de dialyse selon la revendication 5, **caractérisée par** une première conduite d'acheminement (10) comportant une première soupape de commutation (20) et une deuxième conduite d'acheminement (14) comportant une deuxième soupape de commutation (24), dans laquelle les deux soupapes de commutation (20, 24) ont des sections d'ouverture identiques ou différentes.

7. Machine de dialyse selon la revendication 5, **caractérisée en ce que** le système de soupape présente une autre soupape de commutation (120, 124) qui est aménagée parallèlement à la première soupape de commutation (20, 24).

8. Machine de dialyse selon la revendication 7, **caractérisée en ce que** les sections d'ouverture des deux soupapes de commutation (20, 120, 24, 124) du système de soupape respectif ont un rapport de 1:2.

9. Machine de dialyse selon l'une quelconque des revendications 5 à 8, **caractérisée en ce qu'**au niveau de la soupape de commutation (20, 24) ou des soupapes de commutation (20, 120, 24, 124) peuvent être transmises des impulsions d'ouverture de longueurs différentes de préférence à une fréquence qui reste constante.

10. Machine de dialyse selon l'une quelconque des revendications 5 à 8, **caractérisée en ce qu'**au niveau de la soupape de commutation (20, 24) ou des soupapes de commutation (20, 120, 24, 124) peuvent être transmises des impulsions d'ouverture de mêmes longueurs ayant une fréquence différente.

11. Machine de dialyse selon l'une quelconque des revendications 3 à 10, **caractérisée en ce que** dans la conduite principale (6) entre le dispositif de mesure de la conductivité (36) et la pompe (4), un autre dispositif de mesure de la conductivité (38) est aménagé, dont un signal peut être transmis à un agencement de surveillance (40).

12. Machine de dialyse selon l'une quelconque des revendications précédentes, **caractérisée en ce que** dans la conduite principale (6) en amont de l'embouchure (12) est aménagée une soupape d'arrêt commandée de façon électromagnétique.

13. Machine de dialyse selon la revendication 1, **caractérisée en ce que** la soupape de réglage de la pression (28) est une soupape de réglage de la pression réglable, actionnée par la pression.

14. Machine de dialyse selon la revendication 5, **caractérisée en ce que** la soupape de commutation (20, 24) est une soupape de commutation 2/2.

15. Machine de dialyse selon la revendication 7, **caractérisée en ce que** l'autre soupape de commutation (120, 124) est une soupape de commutation 2/2.
